# EUROPEAN PATENT APPLICATION

(11) **EP 3 591 097 A1**
(43) Date of publication of application: **08.01.2020**
(21) Application number: 18181873.3
(22) Date of filing: 05.07.2018
(51) Int. Cl.: C25C 7/00, C25C 5/02, C25C 1/20, A61K 33/38

(54) **METHOD AND PLANT FOR THE PREPARATION OF METALLIC UNCOATED NANOCLUSTERS**

(71) Applicant: Universita' Degli Studi G. D Annunzio Chieti - Pescara, 66100 Chieti (IT)
(72) Inventor: SCOTTI, Luca, 65024 MANOPPELLO (PE) (IT)
(74) Representative: Currado, Luisa

(57) **Abstract**

An electrochemical device characterized in having rotating circular electrodes being useful for the electrochemical synthesis of uncoated metallic nanocluster, the process for the production thereof are disclosed.

## Description

### Technical field

The present invention refers to the field of chemistry technology and in particular to the field of electrochemical process since it refers to a electrochemical device characterized in having rotating circular electrodes being useful for the electrochemical synthesis of uncoated metallic nanocluster, the process for the production thereof.

### Background of the invention - Prior Art

It is known the preparation of Ag nanoclusters by using a conventional three-electrode system composed of a modified glass carbon electrode (CGE) as the working electrode, a platinum wire as the auxiliary electrode, and a saturated calomel electrode (SCE) as the reference electrode. The process includes the polishing of the modified glass carbon electrode with alumina powder, followed by washing and cleaning with ultrapure water and ethanol, respectively; then the modified glass carbon electrode is dried with nitrogen at room temperature. The Ag NCs were in situ electrodeposited on GCE by dynamic potential scanning between 0 and 1.2 V with the three-electrode system in the presence of a conductive solution, consisting of 1 mM AgClO₄ in 2 mL LiClO₄-glycine buffer deaerated by bubbling nitrogen keeping an inert atmosphere during the whole process (Ying Zhou, Yanqing Yu, Yaqin Chai, Ruo Yuan, 2018, Electrochemical synthesis of silver nanoclusters on electrochemiluminescent resonance energy transfer amplification platform for Apo-A1 detection, Talanta 181, 32-37).

Silver nanoclusters have been prepared by situ biosynthesis of oligonucleotide-templated fluorescent silver nanoclusters (AgNCs),using endogenous glutathione (GSH) in Human mesenchymal stem cells (hMSCs) followed by treatment with silver nitrate (AgNO₃) as nanocluster precursors and oligonucleotide of cytosine (C) bases as templates. The biosynthesized Silver nanoclusters AgNCs were used for real-time determination of telomerase activity (Fangyuan Dong, Enduo Feng, Tingting Zheng, Yang Tian, In Situ Synthesized Silver Nanoclusters for Tracking the Role of Telomerase Activity in the Differentiation of Mesenchymal Stem Cells to Neural Stem Cells, 2018, ACS Appl. Mater. Interfaces,10, 2051-2057).

US patent n. US7128816 discloses an apparatus for the preparation of colloidal dispersions of nanoparticles of electrically conducting materials, comprising a reactor comprising at least one static electrode and at least one rotating electrode.

Australian patent application n. AU198931245A disclosed an Electrode assembly for electrochemical reactions including a discoidal or annular rotating electrode for the electrohydrodimerisation of acrylonitrile.

European patent n. EP333364 discloses an electrode assembly including non-permeable, charged, rotating electrode for the electrochemical reaction between a liquid first phase and a liquid organic second phase.

The same inventors already assembled a novel device for the production of stable solutions of spherical uncoated silver nanoparticles (SNPs) in only 10 minutes, comprising a four electrodes cell with silver plate (75 × 28 × 0.5 mm) for the anode and cathode at a distance of 10 mm each other, a power supply, a electronic board to generate 200 V electrical current and 0.5 Hz square wave to switch the polarity between electrodes every second, wherein the power supply keeps constant voltage, current and total electrical power (W), while the electronic board controls the frequency of the square wave to allow the polarity inversion (L. Scotti, G. Angelini, C. Gasbarri, T. Bucciarelli, uncoated negatively charged silver nanoparticles: speeding up the electrochemical synthesis, 2017, Materials Research Express, 4(10), 1-5).

### Technical problem

In view of the findings of the prior art, and in particular considering what disclosed in (L. Scotti, G. Angelini, C. Gasbarri, T. Bucciarelli, uncoated negatively charged silver nanoparticles: speeding up the electrochemical synthesis, 2017, Materials Research Express, 4(10), 1-5), the same inventors investigated the modifications to the device designed for the production of spherical uncoated silver nanoparticles (SNPs) in order to allow the production of uncoated metallic nanoclusters.

The above device presents a very important drawback since oxides deposit on the surface of the electrodes, requiring cleaning or substitution of the electrodes at the end of each synthesis, because oxides deposition negatively affects controlled working parameters.

Moreover the known processes for the synthesis of metal nanoclusters are based on redox reaction in the presence of oxidation and reducing agents.

The aim of the present invention was therefore to avoid the above drawback and design an upgraded device able to work continuously and maintaining the controlled working parameters and reducing inactivity of the device and servicing.

An other aim of the present invention was the set up of a process avoiding the use of oxidation and reducing agents which can negatively affect purity of the final product.

### Object of the invention

The technical problem is therefore solved by providing an electrochemical device 1 comprising a power supply generating square wave electrical current 2 and, an electrolytic cell 3 comprising a main chamber 4 filled with water, and a lid 5, a pumping unit 6, and a filtering unit 7 wherein the lid 5 has at least four passing-through slits 8, each hosting corresponding disc-shaped electrode 9, all the electrodes 9 being journaled around a pivot shaft 10 arranged transversal to said slits 8 and connected to said power supply 2, the lid 7 comprising scraping means 12 having a shape complementary to the edge and to the side faces of said electrodes 9.

Another object of the present invention is a process for the preparation of uncoated metal nanoaggregates by using electrochemical device 1 comprising a power supply generating square wave electrical current 2 and, an electrolytic cell 3 comprising a main chamber 4 filled with water, and a lid 5, a pumping unit 6, and a filtering unit 7 wherein the lid 5 has at least four passing-through slits 8, each hosting corresponding disc-shaped electrode 9, all the electrodes 9 being journaled around a pivot shaft 10 arranged transversal to said slits 8 and connected to said power supply 2, the lid 7 comprising scraping means 12 having a shape complementary to the edge and to the side faces of said electrodes 9 are made of the same metal of the uncoated metal nanocluster to be produced
comprising the following steps
a) adding fresh water in the main chamber;
b) heating the solution at a temperature ranging between 25 °C and 90 °C;
c) activating the electric power supply;
d) rotating the electrodes till the synthesis of metal nanoclusters is concluded
e) continuously filtering the water
f) emptying the main chamber at the end of the process from water containing the uncoated metal nanocluster produced ; and
g) removing the side-products being nanoparticles with a diameter between 200 nm and 300 nm nanoparticles metal nanoclusters from the filtering unit.

Another object of the present invention are the uncoated metal nanoclusters obtained from the process of the invention.

Another object of the present invention is the use of the uncoated metal nanoclusters obtained from the process of the invention as bacteriostatic agent.

Another object of the present invention is the use of the uncoated metal nanoclusters obtained from the process of the invention as bactericide agent.

Further characteristic of the present invention will be clear from the following detailed description with reference to the experimental results presented and the attached drawings.

### Brief Description of the figures

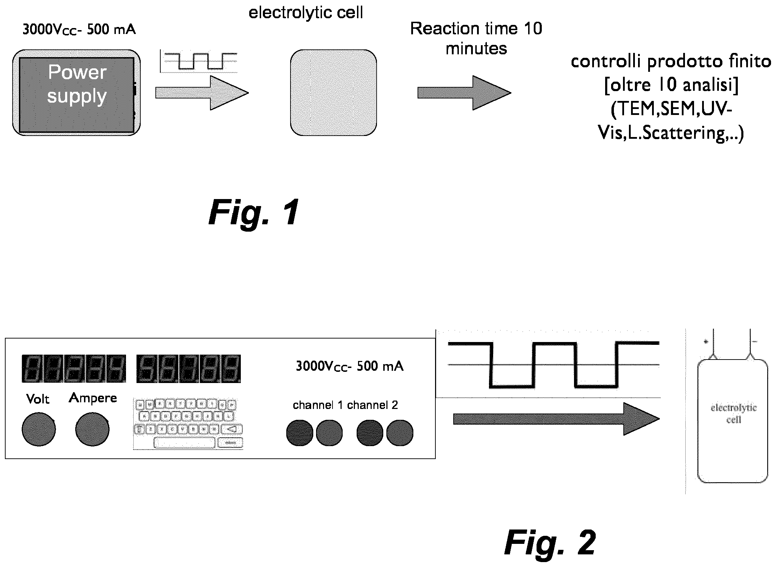

Figure 1 shows the working scheme of the electrochemical device.
Figure 2 shows the working scheme of the power supply together with the electrolytic cell.

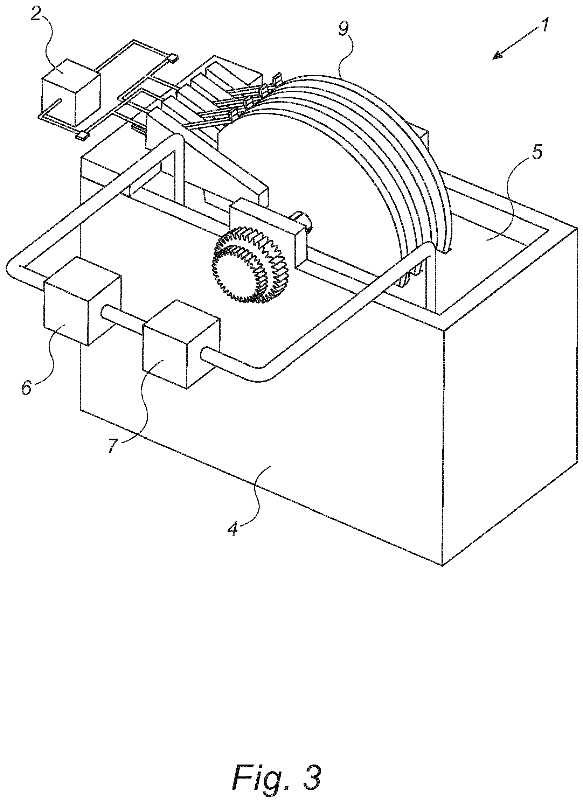

Figure 3 shows the tridimensional representation of the electrochemical device.

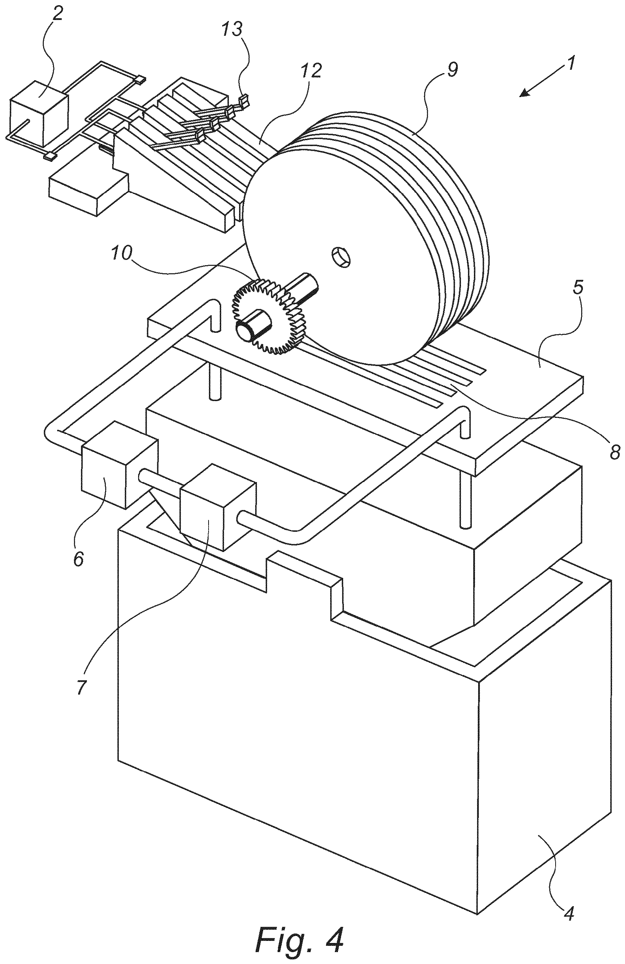

Figure 4 shows the exploded view drawing of the electrochemical device.

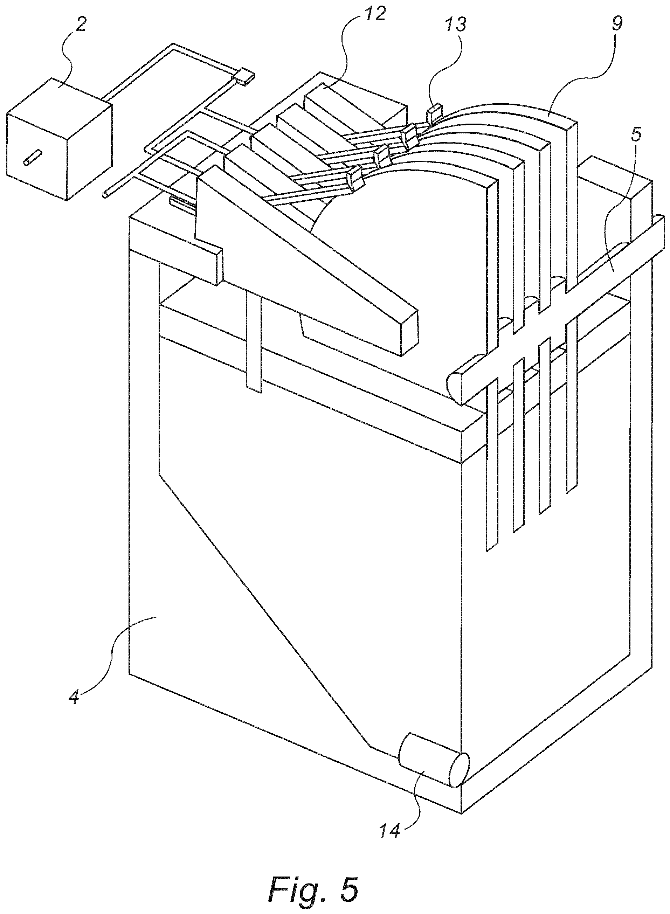

Figure 5 shows the longitudinal section of the of the electrochemical device.

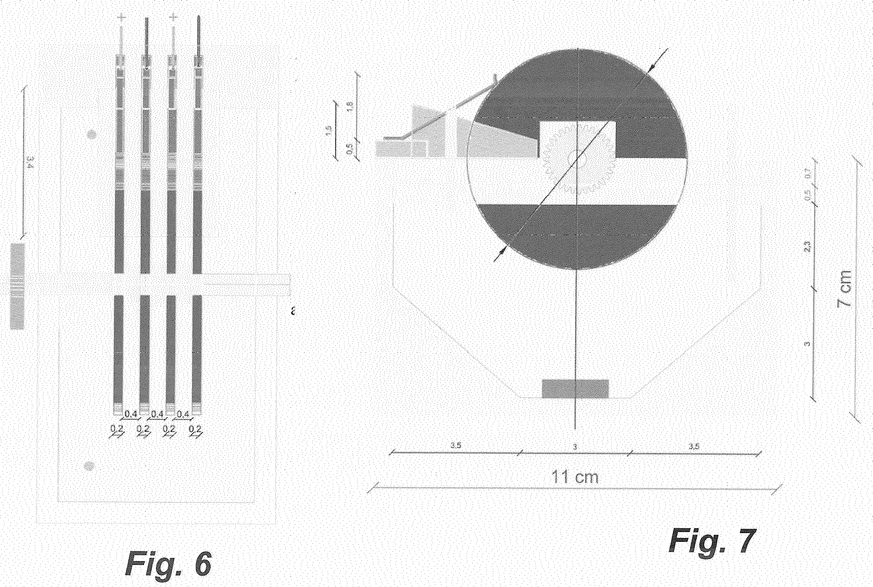

Figure 6 shows the section the lid.
Figure 7 shows plane section of the of the electrochemical device.
Figure 8 shows in graph toxicity on HEK cells and viability of HEK cells after exposure to AgNPs at different doses.
Figure 9 shows in graph shows in graph toxicity on HEK cells and viability of HEK cells after exposure to AgNPs at different doses.

### Detailed description of the Invention

### Definitions

Within the meaning of the present invention uncoated metal nanoaggregates means clusters of metal particles without any coating on their surface having a mean diameter than 5nm.

Wherein metal can be selected from the group consisting of: gold, silver, copper, zinc, iron, cadmium, aluminium.

Within the meaning of the present invention square means non-sinusoidal periodic waveform in which the amplitude alternates at a steady frequency between fixed minimum and maximum values, with the same duration at minimum and maximum.

Object of the present invention is an electrochemical device 1 comprising a power supply generating square wave electrical current 2 and, an electrolytic cell 3 comprising a main chamber 4 filled with water, and a lid 5, a pumping unit 6, and a filtering unit 7 wherein the lid 5 has at least four passing-through slits 8, each hosting corresponding disc-shaped electrode 9, all the electrodes 9 being journaled around a pivot shaft 10 arranged transversal to said slits 8 and connected to said power supply 2, the lid 7 comprising scraping means 12 having a shape complementary to the edge and to the side faces of said electrodes 9.

The main chamber can be additionally equipped with an outlet valve 14 for emptying the main chamber from water.

The main chamber can be additionally equipped with a heating device to heat water.

Preferably the disc-shaped electrode are parallel to each other and perpendicular to the pivot shaft and spaced to each other of a distance ranging from 4 mm to 80 mm, More preferably the distance is 10 mm.

Preferably the scraping means comprises a support connected to the electric power supply and a plurality of brushes and/or bristles disposed in rows 13 inserted between each pair of adjacent electrodes.

Another object of the present invention is a process for the preparation of uncoated metal nanoaggregates by using electrochemical cell device 1 comprising a power supply generating square wave electrical current 2 and, an electrolytic cell 3 comprising a main chamber 4 filled with water, and a lid 5, a pumping unit 6, and a filtering unit 7 wherein the lid 5 has at least four passing-through slits 8, each hosting corresponding disc-shaped electrode 9, all the electrodes 9 being journaled around a pivot shaft 10 arranged transversal to said slits 8 and connected to said power supply 2, the lid 7 comprising scraping means 12 having a shape complementary to the edge and to the side faces of said electrodes 9, and said electrodes 9 are made of the same metal of the uncoated metal nanocluster to be produced, comprising the following steps:
a) adding fresh water in the main chamber;
b) heating the solution at a temperature ranging between 25 °C and 90 °C;
c) activating the electric power supply;
d) rotating the electrodes till the synthesis of metal nanoclusters is concluded
e) continuously filtering the water
f) emptying the main chamber at the end of the process from water containing the uncoated metal nanocluster produced ; and
g) removing the side-products being nanoparticles with a diameter between 200 nm and 300 nm nanoparticles metal nanoclusters from the filtering unit.

Another object of the present invention are the uncoated metal nanoclusters prepared by using by using electrochemical cell device 1 comprising a power supply generating square wave electrical current 2 and, an electrolytic cell 3 comprising a main chamber 4 filled with water, and a lid 5, a pumping unit 6, and a filtering unit 7 wherein the lid 5 has at least four passing-through slits 8, each hosting corresponding disc-shaped electrode 9, all the electrodes 9 being journaled around a pivot shaft 10 arranged transversal to said slits 8 and connected to said power supply 2, the lid 7 comprising scraping means 12 having a shape complementary to the edge and to the side faces of said electrodes 9, and said electrodes 9 are made of the same metal of the uncoated metal nanocluster to be produced, comprising the following steps:
a) adding fresh water in the main chamber;
b) heating the solution at a temperature ranging between 25 °C and 90 °C;
c) activating the electric power supply;
d) rotating the electrodes till the synthesis of metal nanoclusters is concluded
e) continuously filtering the water
f) emptying the main chamber at the end of the process from water containing the uncoated metal nanocluster produced ; and
g) removing the side-products being nanoparticles with a diameter between 200 nm and 300 nm nanoparticles metal nanoclusters from the filtering unit.

The temperature of heating in step a) varies depending on the type of nanocluster to be prepared.

The working parameters of the power supply depend on the type of final product to be prepared and also to the dimension of the device and of the electrolytic cell which can be scaled up to a higher volume requiring more energy to be applied.

In step g) the side-products being nanoparticles with a larger diameter, between 200 nm and 300 nm, are removed by the filtering unit, the dimension of the removed particles depends on the pore size of the filter used.

Preferably in step c) the electric power supply generates a square wave alternate current with a voltage ranging between 3 V and 3000 V, with an intensity ranging between 100 mA and 4 A, and with a frequency ranging between 0,01 Hz and 1 Hz.

Preferably the time for completing of the synthesis of metal nanoclusters is from 1 to 25 minutes.

Preferably the power supply generating electrical current and square wave can be set in time intervals from seconds to minutes.

Preferably the electrodes are made of 99,9% the same metal constituting the uncoated nanoaggregate to be prepared, more preferably of gold, silver, copper, most preferably of silver.

Preferably the at least four electrodes are placed at a distance ranging from 4 mm to 80 mm, most preferably the distance is of 10 mm.

More preferably the square wave is of 0.5 Hz to switch the polarity between electrodes every second and of frequency of the square wave to allow the polarity inversion.

Preferably the cleaning element comprises a support 5 connected to the power supply and a plurality of brushes and/or bristles disposed in lines 16 to insert between each electrodes of circular shape to fit within and continuously cleaning the surfaces of the electrodes.

Preferably the cleaning element is made of rubber.

Preferably in step a) water is ultrapure water

More preferably in step b) the temperature is of 70°C

More preferably in step c) the power supply is at 220 V alternating current in 200 V direct current with 200 mA and square wave with 1Hz of frequency

More preferably the time for completing of the synthesis of metal nanoclusters is of 10 minutes.

Preferably the uncoated metal nanocluster is uncoated nanocluster of gold, silver, copper, more preferably are of silver.

Another object of the present invention is the use of the uncoated metal nanoclusters obtained from the process of the invention as bacteriostatic agent.

Another object of the present invention is the use of the uncoated metal nanoclusters obtained from the process of the invention as bactericide agent.

Preferably the bactericide agent and/or bacteriostatic agent is used against gram negative and/or gram positive bacteria.

Preferably gram negative bacteria are selected from the group consisting of

Preferably gram positive bacteria are selected from the group consisting of

### Examples

A pilot plant for synthesis of silver uncoated metal nanoclusters was developed having a four electrodes cell with silver plate (75 × 28× 0,5 mm) for the anode and cathode (distance 10 mm). The power supply (model Pharmacia LKB GPS 200/400, Japan LKB Pharmacia) and a home made electronic board were employed to generate 200 V electrical current and 0.5 Hz square wave to switch the polarity between electrodes every second. The power supply is able to keep constant voltage (V), current (A) and total electrical power (W), while the electronic board controls the frequency of the square wave to allow the polarity inversion. Then the silver solution was filtered through 0.22 µm pore size Millipore filters and the UV-vis spectra were recorded in the spectral range 300-600 nm by using a spectrophotometer Jasco 7800. Dynamic laser light scatter- ing and zeta-potential measurements were carried out at 25 °C using 90PLUS BI-MAS (Brookhaven) equipped with digital correlator at a scattering angle of 90°, with a 35 mW He-Ne laser at the wavelength of 660 nm. The particles concentration was measured using Nanosight (Malvern, UK). TEM (JASCO, Japan) images were taken at 75kV (ZEISS 109 equipped with Gatan-Orius SC200W-Model 830.10W TEM CCD Camera) after evaporation of a drop of diluted solution (1:50) of SNPs on 300 mesh formvar coated nickel grids. FE-SEM images and microanalysis were performed on the powder from the complete evaporation under nitrogen flow of the SNPs solution at 40 °C (SEM Sigma 300 Zeiss; microanalysis Quantax-200 Bruker, Germany). Pure water-RPE (<0.1 mS cm⁻¹; all residual metal <0.01 ppm) was purchased by Carlo Erba (Italy) and metal for electrodes (Silver 99.9%) from local jewelry. The current density of the cell was 3 mA cm² at the beginning of the synthesis and was increased up to 4 mA cm² till the end of the process (total run 10 min). The temperature of solution was kept constant to 80 ± 0.5 °C for every reaction time and the process was monitored with an amperometric instrument.

Ultra nanocluster in the range 0.4-1.5 nm and in concentration up to 0.63 mM were synthesized. The electrochemical synthesis was performed in 10 minutes. The values of zeta-potential value registered demonstrated the good stability of the nanoclusters obtained.

Chemical and physical characterization was performed by using DLS Light scattering for particles size, poly-dispersion and zeta potential, TEM Transmission Electron Microscopy for morphology, distribution and particles size, UV-Vis for Plasmodic emission (max absorbance at 400 nm), FE-SEM for elementary analysis , morphology, particles size, MALDI-TOF for cluster analysis and mass spectroscopy, ICP-MS for quantitative analysis of Silver solution.

The final solution are filtered on 0,22 um membrane and capped under nitrogen in dark glass bottle, being stable for 6 months (checked zeta potential , particles size and poly-dispersion parameters).

matrix-assisted laser desorption/ionization (MALDI) was performed on the nanoclusters above obtained.

1 uL of AgNPs aqueous solution at 20 ppm was deposited on the MALDI target and added 0.5 uL of trifluoroacetic acid 0.1%. After air drying the target was inserted into the mass spectrometer and acquired. The analysis was performed on the Autoflex Speed TOF/TOF mass spectrometer (Bruker-Daltonics GmbH, Bremen, Germany) equipped with a SmartBeam II 1kHz laser, in Reflectron Positive (RP) mode, in the range 20-1,300 Da. The instrument was controlled through Flexcontrol 3.4.135.0 software. Cubic enhanced external calibration of the TOF tube was performed before the acquisition using CHCA adducts mixed with Bradykinin (1-7) and Angiotensin II. The following monoisotopic masses were used for calibration: K+ 38.9637, CHCA [M+H-H2O]+ 172.0399, CHCA [M+H]+ 190.0504, CHCA [M+Na]+ 212.0324, CHCA [2M+H-CO2]+ 335.1032, CHCA [2M+H]+ 379.0930, Bradykinin(1-7) [M+H]+ 757.3991 and Angiotensin II [M+H]+ 1046.5418. The instrumental voltages were set as follows: 19.00 kV and 16.70 kV for the ion source 1 and 2, respectively, 8.55 kV for the lens and 21.00 kV and 9.60 kV for reflector 1 and 2, respectively. Pulsed ion extraction time was set to 130 ns. Laser fluency was kept very low to avoid unwanted fragmentation of sample clusters and adducts. The power attenuator was adjusted before the acquisition to maximize resolution, and a laser frequency of 100 Hz was used. 5,000 to 15,000 total shots were summed for each sample spot.

The cromatoghaphy profile indicated the presence of silver in metal form (oxidative state 0) with the following values: Ag 106.905 (Mw) ione +1 ; Ag2 215.812 (Mw) ion +1 ; Ag3 322,750 (Mw) ion +1 ; Ag5 540.582 (Mw) ion +1 ; Ag7 754.425 (Mw) ion +1 ; Ag9 970.252 (Mw) ion +1 ; Ag11 1186.007 (Mw) ion +1; and corresponding with calculate theoretical value. There were no evidence of impaired silver metal and other oxidative state of silver. There were only odd multiples of atoms.

The silver uncoated nanoclusters were tested as bacteriostatic agent and bactericide agent.

AgNPs was tested for toxicity in G. mellonella, and results are shown in the following table 1, Wherein tobramicin is used as standard.

**Table 1**

| Time (h) | PBS (10 µL) | H₂O (10 µL) | H₂O (20 µL) | AgNPs (3,4 µg/ml) | AgNPs (6,8 µg/ml) | TOB (4 µg/ml) |
|---|---|---|---|---|---|---|
| 0 | 100 | 100 | 100 | 100 | 100 | 100 |
| 24 | 96,6 | 96,6 | 95 | 100 | 91,6 | 100 |
| 48 | 95 | 95 | 90 | 98,3 | 90 | 98,3 |
| 72 | 95 | 95 | 90 | 98,3 | 88,3 | 98,3 |
| 96 | 95 | 95 | 90 | 98,3 | 85 | 98,3 |

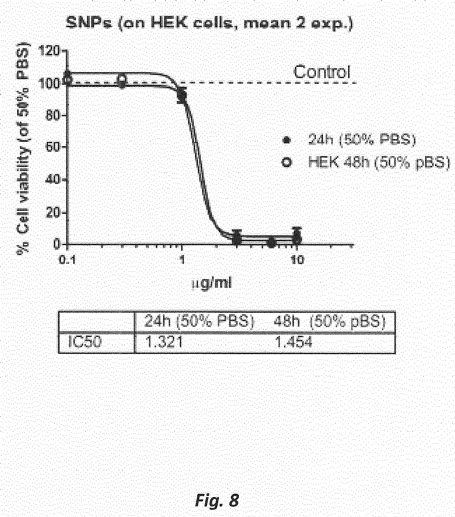

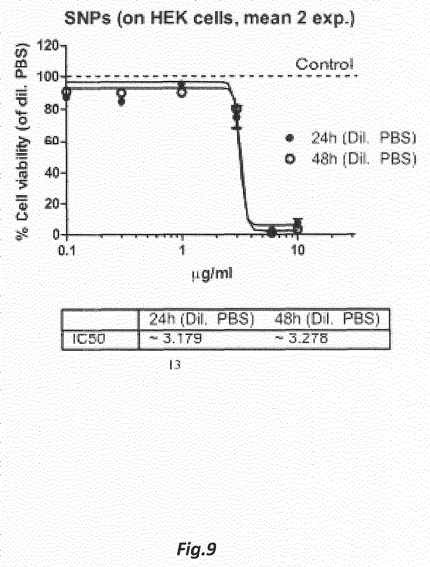

The above results underline that over the study period, the mortality rate ranged from 0 to 1.7% and from 8.3 and 11,7 % following exposure to AgNPs respectively at 3.4 and 6.8 µg/ml
AgNPs was tested in vitro on Human embryonic kidney cells (HEK cells) for toxicity and the results of cell viability are reported in figure 8, and 9.

## Claims

1. Electrochemical cell device (1) comprising a power supply generating square wave electrical current (2) and, an electrolytic cell (3) comprising a main chamber (4) filled with water, and a lid (5), a pumping unit (6), and a filtering unit (7), wherein the lid (5) has at least four passing-through slits (8), each hosting corresponding disc-shaped electrode (9), all the electrodes (9) being journaled around a pivot shaft (10) arranged transversal to said slits (8) and connected to said power supply (2), the lid (7) comprising scraping means (12) having a shape complementary to the edge and to the side faces of said electrodes (9).

2. Electrochemical cell device (1) according to claim 1 wherein the electrodes (9) are parallel to each other and perpendicular to the pivot shaft and spaced to each other of a distance ranging from 4 mm to 80 mm.

3. Electrochemical cell device (1) according to claim 3 wherein the distance is 10 mm.

4. Electrochemical cell device (1) according to claim 1 wherein said scraping means (12) comprises a support connected to the electric power supply (2) and a plurality of brushes and/or bristles disposed in rows (13) inserted between each pair of adjacent electrodes (9).

5. Process for the preparation of uncoated metal nanoaggregates by using electrochemical cell device (1) comprising a power supply generating square wave electrical current (2) and, an electrolytic cell (3) comprising a main chamber (4) filled with water, and a lid (5), a pumping unit (6), and a filtering unit (7) wherein the lid (5) has at least four passing-through slits (8), each hosting corresponding disc-shaped electrode (9), all the electrodes (9) being journaled around a pivot shaft (10) arranged transversal to said slits (8) and connected to said power supply (2), the lid (7) comprising scraping means (12) having a shape complementary to the edge and to the side faces of said electrodes (9), and said electrodes (9) are made of the same metal of the uncoated metal nanocluster to be produced, comprising the following steps:
a) adding fresh water in the main chamber;
b) heating the solution at a temperature ranging between 25 °C and 90 °C;
c) activating the electric power supply;
d) rotating the electrodes till the synthesis of metal nanoclusters is concluded
e) continuously filtering the water
f) emptying the main chamber at the end of the process from water containing the uncoated metal nanocluster produced ; and
g) removing the side-products being nanoparticles with a diameter between 200 nm and 300 nm nanoparticles metal nanoclusters from the filtering unit.

6. Process for the preparation of uncoated metal nanoaggregates according to claim 5, wherein the freshwater is ultrapure water.

7. Process for the preparation of uncoated metal nanoaggregates according to claim 5, wherein in step b) the temperature is 70°C

8. Process for the preparation of uncoated metal nanoaggregates according to claim 5, wherein in step c) the power supply generates a square wave alternate current with a voltage ranging between 3 V and 3000 V, with an intensity ranging between 100 mA and 4 A, and with a frequency ranging between 0,01 Hz and 1 Hz.

9. Uncoated metal nanoclusters prepared by using an electrochemical cell device (1) comprising a power supply generating square wave electrical current (2) and, an electrolytic cell (3) comprising a main chamber (4) filled with water, and a lid (5), a pumping unit (6), and a filtering unit (7) wherein the lid (5) has at least four passing-through slits (8), each hosting corresponding disc-shaped electrode (9), all the electrodes (9) being journaled around a pivot shaft (10) arranged transversal to said slits (8) and connected to said power supply (2), the lid (7) comprising scraping means (12) having a shape complementary to the edge and to the side faces of said electrodes (9) and said electrodes (9) are made of the same metal of the uncoated metal nanocluster to be produced, in a process comprising the following steps:
a) adding fresh water in the main chamber;
b) heating the solution at a temperature ranging between 25 °C and 90 °C;
c) activating the electric power supply;
d) rotating the electrodes till the synthesis of metal nanoclusters is concluded
e) continuously filtering the water
f) emptying the main chamber at the end of the process from water containing the uncoated metal nanocluster produced ; and
g) removing the side-products being nanoparticles with a diameter between 200 nm and 300 nm nanoparticles metal nanoclusters from the filtering unit.

10. Uncoated metal nanoclusters according to claim 10, wherein the metal is silver.

11. Uncoated metal nanoclusters according to claim 10, for use as bacteriostatic agent.

12. Uncoated metal nanoclusters according to claim 10, for use as bactericide agent.
